# EUROPEAN PATENT SPECIFICATION

(11) **EP 3 479 780 B1**
(45) Date of publication and mention of the grant of the patent: **29.06.2022**
(21) Application number: 18204373.7
(22) Date of filing: 05.11.2018
(51) Int. Cl.: A61B 17/072

(54) **MULTI-LAYER TISSUE THICKNESS COMPENSATOR COMPRISING RESILIENT AND SACRIFICIAL COLLAPSIBLE LAYERS**
MEHRSCHICHTIGER GEWEBEDICKENKOMPENSATOR MIT ELASTISCHEN UND ZUSAMMENSCHIEBBAREN OPFERSCHICHTEN
COMPENSATEUR DE L'ÉPAISSEUR D'UN TISSU MULTICOUCHES COMPRENANT DES COUCHES REPLIABLES RÉSILIENTES ET SACRIFICIELLES

(30) Priority: 06.11.2017 US 201715804280
(43) Date of publication of application: 08.05.2019
(73) Proprietor: Ethicon LLC, Guaynabo 00969 (PR)
(72) Inventor: KRIKSUNOV, Leo B., Somerville, NJ 08876 (US); TANNHAUSER, Robert J., Somerville, NJ 08876 (US)
(74) Representative: Carpmaels & Ransford LLP

(56) References cited:
- EP-A2- 2 644 126
- EP-A2- 3 143 947
- EP-A2- 3 150 138
- US-A1- 2012 241 491
- US-A1- 2012 318 844

## Description

### BACKGROUND

The present examples relate to stapling instruments and, in various embodiments, to a surgical stapling instrument for producing one or more rows of staples.

A stapling instrument can include a pair of cooperating elongate jaw members, wherein each jaw member can be adapted to be inserted into a patient and positioned relative to tissue that is to be stapled and/or incised. In various embodiments, one of the jaw members can support a staple cartridge with at least two laterally spaced rows of staples contained therein, and the other jaw member can support an anvil with staple-forming pockets aligned with the rows of staples in the staple cartridge. Generally, the stapling instrument can further include a pusher bar and a knife blade which are slidable relative to the jaw members to sequentially eject the staples from the staple cartridge via camming surfaces on the pusher bar and/or camming surfaces on a wedge sled that is pushed by the pusher bar. In at least one embodiment, the camming surfaces can be configured to activate a plurality of staple drivers carried by the cartridge and associated with the staples in order to push the staples against the anvil and form laterally spaced rows of deformed staples in the tissue gripped between the jaw members. In at least one embodiment, the knife blade can trail the camming surfaces and cut the tissue along a line between the staple rows. Examples of such stapling instruments are disclosed in U.S. Pat. No. 7,794,475, entitled SURGICAL STAPLES HAVING COMPRESSIBLE OR CRUSHABLE MEMBERS FOR SECURING TISSUE THEREIN AND STAPLING INSTRUMENTS FOR DEPLOYING THE SAME, U.S. Patent Application No. 13/433,163 entitled METHODS FOR FORMING TISSUE THICKNESS COMPENSATOR ARRANGEMENTS FOR SURGICAL STAPLES, U.S Pat. No. 9,113,865 entitled STAPLE CARTRIDGE COMPRISING A LAYER, U.S. Pat. No. 9,433,419 entitled TISSUE THICKNESS COMPENSATOR COMPRISING A PLURALITY OF LAYERS.

The foregoing discussion is intended only to illustrate various aspects of the related art in the field of the invention at the time, and should not be taken as a disavowal of claim scope.

While various kinds of devices and methods have been made and used, it is believed that no one prior to the inventor(s) has made or used the invention described in the appended claims.

A tissue thickness compensator that generally comprises a biocompatible material, a first component, and a second component, wherein the first component and second component form a reaction product to expand the tissue thickness compensator is described in US 2012/241491 A1.

An apparatus including a housing, a platform, and a buttress assembly is described in EP 3 143 947 A2.

A compressible adjunct for use with a surgical instrument including a staple cartridge including a first biocompatible layer, a second biocompatible layer spaced apart from the first biocompatible layer, and a plurality of supporting pillars extending between the first biocompatible layer and the second biocompatible layer is described in EP 3 150 138 A2.

A staple cartridge assembly for use with a surgical stapler, the assembly having a cartridge body having a support portion with a plurality of staple cavities with openings is described in US 2012/318844 A1.

A surgical stapling instrument including a first jaw that supports a plurality of surgical staples and a second jaw that is movable relative to the first jaw is described in EP 2 644 126 A2.

### BRIEF DESCRIPTION OF THE DRAWINGS

The present invention provides a fastner cartridge assembly for a surgical instrument, the fastener cartridge assembly comprising: a) a tissue thickness compensator (200) compressible to a strain level, the tissue thickness compensator comprising: i.a resilient compensation layer (130) having a first pressure-compression curve; ii. a collapsible compensation layer (120) adjacent said resilient compensation layer, said collapsible compensation layer having a second pressure-compression curve; and b) a fastener (10) moveable between an initial position and a fired position, wherein said fastener is configured to compress at least a portion of said tissue thickness compensator when fastener is moved to said fired position, characterised in that a first slope of said second pressure-compression curve is greater than a first slope of said first pressure-compression curve at a low compression and a second slope of said second pressure-compression curve is less than a second slope of said first pressure-compression curve at a compression higher than said low compression.

While the specification concludes with claims which particularly point out and distinctly claim this technology, it is believed this technology will be better understood from the following description of certain examples taken in conjunction with the accompanying drawings, in which like reference numerals identify the same elements and in which:
FIGs. 1A-1C illustrate an example of a prior art collapsible tissue compensator.
FIGs. 2A-2G illustrate examples of a two-layer construct comprising a sacrificial collapsible layer and a resilient layer.
FIGs. 3A-3C illustrate a schematic view of an example tissue thickness compensator having a multilayer construct.
FIG. 4 illustrates a schematic plot of pressure vs. compression for compressed materials as well as of one example of a resilient layer and sacrificial layer.
FIGs. 5A-5F illustrate configurations of a tissue compensator using one or more examples of a construct described herein.
FIG. 6 illustrates an example collapsible layer comprising a multi-layer construct.
FIG. 7 illustrates an example collapsible layer collapsing under compression.
FIG. 8 illustrates an example collapsible layer comprising a micro molded or micro-machined construct layer.
FIG. 9 illustrates an example collapsible layer 120 comprising a fluid-filled shell, and/or more than one fluid-filled shells therein.
FIG. 10 illustrates a staple cartridge having an example tissue compensator attached.
FIG. 11 illustrates other example geometries of layers that can be used in a tissue compensator.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the person of ordinary skill in the art will readily appreciate that the various methods and devices disclosed herein can be used in numerous surgical procedures and applications including, for example, in connection with open surgical procedures. As the present Detailed Description proceeds, those of ordinary skill in the art will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongated shaft of a surgical instrument can be advanced.

Many of the above-listed patent applications disclose various layers which are used in connection with a staple cartridge. When staples are deployed from the staple cartridge, the staples can capture at least one layer and implant the layer, or layers, against the tissue. Provided below is a brief description of a surgical stapling system. The staple cartridges and the layers disclosed herein can be used with this surgical stapling system and/or any suitable stapling system.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

Various embodiments of tissue thickness compensators having sacrificial layers are disclosed. A tissue thickness compensator of this design has at least one resilient layer and at least one sacrificial collapsible layer. The sacrificial layer collapses reducing its thickness when the tissue compression is high enough to damage tissue. The sacrificial collapsible layer thus decreases the over-compression of the resilient layer, alleviating undesirable excessive compression of tissue.

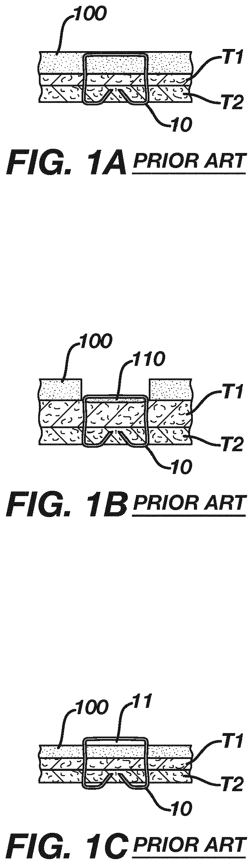

Figures 1A, 1B, and 1C illustrate a prior art tissue collapsible tissue compensator 100. Figure 1A shows a staple 10, which can be a surgical staple or a fastener, implanted into tissue layers T1 and T2. Surgical staple 10 can have a formed height "h" that exceeds the optimum to compress tissue layers T1 and T2 to a compression level that prevents leakage. Adding a tissue thickness compensator 100 compresses tissue layers T1 and T2 to the optimum level to prevent leakage and promote tissue connectivity and healing.

However, choosing the proper tissue compensator thickness can prove difficult. For example, a tissue thickness compensator that is too thick for the given tissue can compress the tissue to a higher than optimum compression level when staple 10 is applied. Figure 1B illustrates a staple 10 having a formed height "h" that is less than the optimum formed height for the combined thicknesses of the tissue thickness compensator 100 and the tissue layers T1 and T2. The amount of pressure applied by the crown 110 of staple 10, and through the compensator to tissue layers T1 and T2 may thus result in tissue ischemia and necrosis. At the other extreme, Figure 1C illustrates tissue compensator 100 that is not thick enough to compensate for thin tissue layers T1 and T2. Pressure applied to the tissue may be lower than optimum, or even, as demonstrated in Figure 1C, the formed staple height "h" may be greater than the combined thickness of tissue compensator 100 and tissue layers T1 and T2. Low pressure between the tissue thicknesses can result in leakage, insufficient joining of tissue, and irritation.

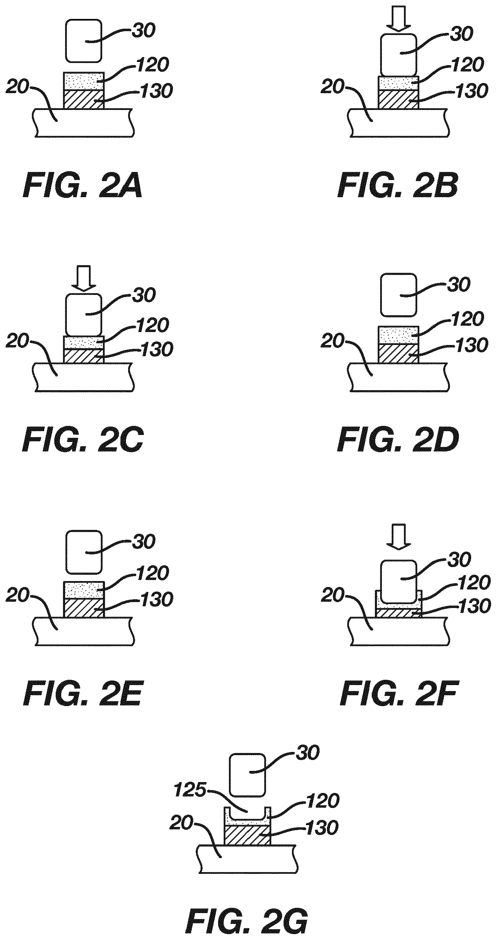

A multiple-layer tissue compensator with at least one sacrificial collapsible layer alleviates the stapling issues illustrated in Figure 1. Figures 2A through 2G illustrate embodiments of a two-layer construct comprising a sacrificial collapsible layer 120 and a resilient layer 130. In this construct, the collapsible layer 120 comprises a material that is relatively rigid at low stress levels, while having high compressibility at higher stress levels. In use, the stress that the material is exposed to is directly related to the force applied to the material divided by the area over which that force is applied. For instance, the stress on the material directly under the crown 110 of the staple 10 is the force that the staple 10 applies to the foam divided by the projected area of the crown 110 onto the material. The force applied to the material is also directly related to the amount of deflection to the material. As previously mentioned, the materials of the sacrificial layer 120 and the resilient layer 130 exhibit a deflection-force curve that is characteristic of the material. Therefore the sacrificial layer 120 and the resilient layer 130 exhibit a reaction force which is proportional to the amount that those materials are compressed. Therefore the stress that a given material is exposed to is greater when applied by a staple 10 where the formed staple height "h" is small than that when applied by a staple 10 where the formed staple height "h" is large. This is because the material becomes more highly deflected by the staple 10 with the smaller formed staple height "h", hence the reaction force of the material is higher and thus the stress is higher. The material of collapsible layer 120 may or may not return to its original height after removal of a compression force. An example of such a material is Surgifoam Absorbable Gelatin Sponge material, available from Johnson and Johnson in Sommerville, NJ. The Surgifoam Absorbable Gelatin Sponge material is made from oxidized cellulose. Other suitable materials may be, but are not limited to, Poly(Lactide-co-Glycolide), Polycaproplactone, or poly-3-hydroxobutyrate or silicone, polyisoprene, and rubber. In various embodiments, the synthetic polymers may comprise expanded polytetrafluoroethylene (ePTFE), commercially available from W. L. Gore & Associates, Inc. under the trade designation GORE-TEX Soft Tissue Patch and co-polyetherester urethane foam commercially available from Polyganics under the trade designation NASOPORE.

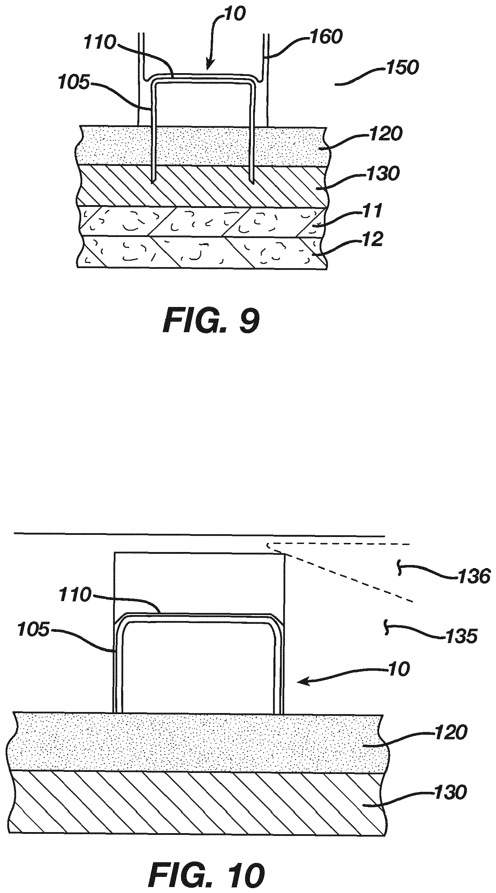

In one embodiment, the sacrificial layer 120 and the resilient layer 130 may be held together by the legs 105 of staple 10. In this embodiment, the legs 105 of staple 10 are of an unfired length such that when the crown 110 of staple 10 is contacting the sacrificial layer 120, the legs 105 extend through the remainder of sacrificial layer 120 and at least partially through the resilient layer 130 as shown in FIG 9. Alternatively, sacrificial layer 120 and resilient layer 130 may be bonded together by an appropriate adhesive, such as, for example, an aliphatic ester homopolymer or a cyanoacrylate or they may be joined by mechanical means such as heat staking of the two materials together. Other embodiments include other methods of mechanical joining such as riveting, sewing, or other mechanical joining means known in the art.

Figures 2A and 2B represent schematically the application of a light force, resulting in a low compression pressure on the construct of sacrificial layer 120 and resilient layer 130. In Figure 2A, the construct rests on a support 20. A compression member 30 is displayed above the construct. Moving compression member 30 to apply a light force on the construct, as shown in Figure 2B, compresses resilient layer 130 while leaving sacrificial layer 120 relatively uncompressed. Removing the light force on the construct by lifting compression member 30 allows resilient layer 130 to return to its pre-compression thickness. The construct also returns to its pre-compression thickness.

Figures 2C and 2D represent schematically application of a moderate force on the construct, resulting in a moderate compression pressure. In Figure 2C, compression member 30 applies a moderate force causing both resilient layer 130 and sacrificial layer 120 to compress. Removing the moderate force from the construct by lifting compression member 30 allows resilient layer 130 and sacrificial layer 120 to return to its pre-compression thickness as shown in FIG 2D.

Figures 2E, 2F, and 2G represent schematically application of a high force on the construct, causing compression pressure in sacrificial layer 120 to climb above the level at which sacrificial layer 120 collapses. Figure 2E shows the construct in the uncompressed state. Figure 2F shows application of the high force and Figure 2G shows the removal of the high force. The high force removal leaves sacrificial layer 120 in the construct of Figure 2G thinner in the collapsed area 125 where compression was applied. However, resilient layer 130 has substantially regained its original thickness. As a result, the overall thickness of the construct within the collapsed area 125 is less than the original thickness before an application of the high compression force.

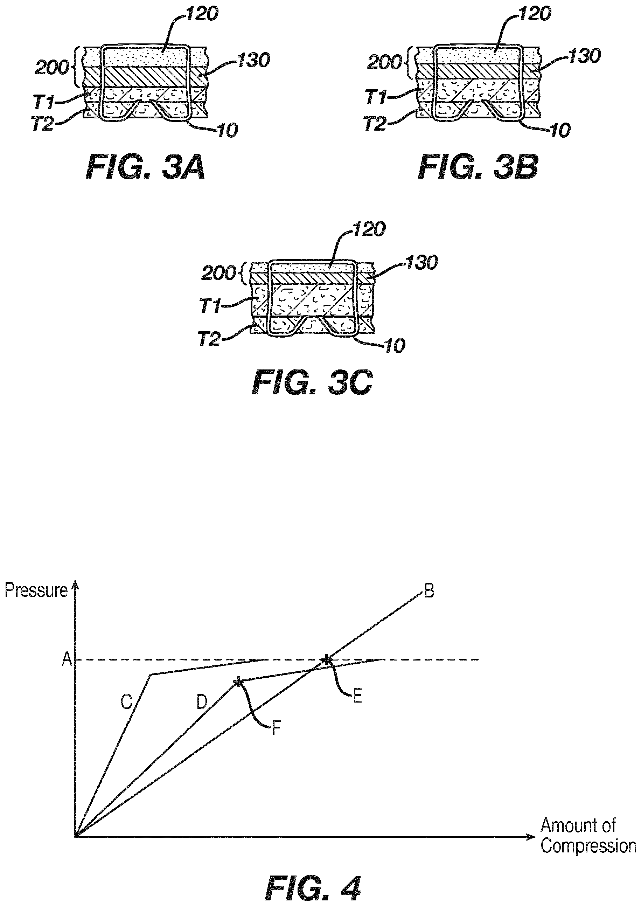

Figures 3A, 3B, and 3C show schematically a tissue thickness compensator 200 having a multilayer construct. Compensator 200 comprises a sacrificial layer 120 and a resilient layer 130. Sacrificial layer 120 is collapsible, and an adhesive or other means described above may attach sacrificial layer 120 to resilient layer 130. Compensator 200 compensates for any varying tissue thicknesses of tissue layers T1 and T2. Surgical staple 10 holds tissue layer T1, tissue layer T2, and compensator 200 in compression at a compression pressure. The amount of this compression pressure varies according to the formed height "h" of staple 10. The compression pressure can also vary as a result of varying thicknesses of tissue layer T1, tissue layer T2, sacrificial layer 120, and resilient layer 130. The compression pressure may also vary as a result of varying compressive properties of tissue layer T1, tissue layer T2, sacrificial layer 120, and resilient layer 130. Surgical staple 10 may be applied to compensator 200 and to tissue layer T1 and tissue layer T2 by any number of surgical staplers, among them may be, for example, Echelon Flex^{™} Powered ENDOPATH^{®} Stapler of any size and length sold by Ethicon in Cincinnati, OH.

Figure 3A demonstrates the usefulness of compensator 200 for thin tissue layers T1 and T2. Such tissues may be in lung parachamyal tissue, for example. In Figure 3A, tissue layer T1 and tissue layer T2 are very thin compared to the formed height of staple 10. Therefore, without a compensator 200, tissue layer T1 and tissue layer T2 would be very lightly compressed or even uncompressed. Compensator 200, placed between the crown 110 of staple 10 and the tissue layers T1 and T2, is slightly compressed under low stress. However, the pressure on tissue layers T1 and T2 is above that needed to promote healing and sealing, and less than the pressure that would cause tissue damage. At low stress, sacrificial layer 120 is substantially rigid and compresses very little. Resilient layer 130 is compressible at low stress and moves elastically under the low stress.

Figure 3B demonstrates compensator 200 used in a situation in which at least one of tissue layer T1 and tissue layer T2 are thicker than tissue layers shown in Figure 3A. In the scenario of Figure 3B, moderate compression pressure will resiliently compress both sacrificial layer 120 and resilient layer 130. However, both sacrificial layer 120 and resilient layer 130 will return substantially to their original uncompressed thickness if staple 10 is removed. In this case both the sacrificial layer 120 and the resilient layer 130 are only compressed within their elastic range, which allows them to return to their original size when pressure is released from their surfaces or to partially return to their original height depending on the amount of pressure being applied to their surfaces.

Figure 3C demonstrates compensator 200 used in a situation in which at least one of tissue layer T1 and tissue layer T2 are significantly thicker than that shown in Figure 3A. In the scenario of Figure 3C, staple 10 will exert enough pressure on sacrificial layer 120 to cause it to permanently collapse. At the collapsing pressure, additional compression of sacrificial layer 120 occurs with very little or no added pressure. Therefore, the pressure applied to tissue layer T1 and tissue layer T2 in the scenario of Figure 3C is not much greater than the moderate compression pressure applied in the scenario of Figure 3B. Compensator 200 reduces in thickness because of the compression pressure applied by staple 10 and the tissue layers, sacrificial layer 120 compresses much more than resilient layer 130. Resilient layer 130, at high pressures, has less compressibility than sacrificial layer 120. After compensator 200 receives a high compression force, sacrificial layer 120 can be permanently compressed and can keep its thickness if compression force is removed. In one embodiment, the compression properties of resilient layer 130 and sacrificial layer 120 are selected such that resilient layer 130 compresses to nearly a solid height prior to any compression in sacrificial layer 120. With respect to the above, the solid height of a layer is characterized in that the material of the layer is compressed to the point that the material reacts to additional compression forces like an incompressible solid. In a second embodiment, the compression properties of sacrificial layer 120 and resilient layer 130 are selected such that sacrificial layer 120 begins to compress after resilient layer 130 experiences some amount of compression, but before resilient layer 130 reaches its solid height. In yet another embodiment the sacrificial layer 120 experiences non-elastic (permanent) compression before or after resilient layer 130 reaches its solid height.

Figure 4 shows a schematic plot of pressure vs. compression for compressed materials as well as of one embodiment of a resilient layer 130 and sacrificial layer 120.

Dotted line A indicates a threshold pressure above which tissue damage can result. Tissue damage can result from ischemia. Pressures typically useful for tissue approximation can be, for example, about 88 kilopascals (nine gram-force per square millimeter). At pressures below this, the tissue may not be well approximated enough to heal or to maintain a seal. At the pressures above this the tissue may be damaged through ischemia which would also negatively impact the ability of the tissue to heal.

Line B schematically illustrates, in a simplified linear way, a pressure-deflection curve of tissue thickness compensator using a foam resilient layer as discussed in the embodiments above. In Figure 4, resilient layer 130 alone becomes over-compressed in the areas of thicker tissue, resulting in undesirable excessive compression force at higher amounts of compression shown at point E. Typical resilient foams, such as, for example, lyophilized Poly (Lactide co-Glycolide) (PLGA), have a pressure-deflection curve that increases with increasing pressure. As shown in figure 4, this pressure-deflection curve can increase linearly or in some cases the curve may increase exponentially, logarithmically or by some other function. In cases as described above, where the material reaches a solid height, the pressure deflection curve may also become vertical at that point.

Line C schematically illustrates, in a simplified linear way, a pressure-deflection curve of a sacrificial layer 120. Typical sacrificial materials have a relatively high stiffness at low pressures, reflected by the steeper slope of the curve at low pressures in Figure 4. At higher pressures, the collapsible material collapses, and the pressure vs deflection curve becomes very low, illustrated schematically in Figure 4 by a change in the slope of line C at point F to a very low slope. Thus, at higher pressures, sacrificial layer 120 collapses and compresses a large distance for a small change in pressure.

Line D schematically illustrates, in a simplified way, a pressure-compression curve created from a combination of a resilient layer 130 having the properties of curve B and a sacrificial layer 120 having the properties of curve C. In embodiments of a multilayer tissue thickness compensator, presence of a sacrificial layer 120 lowers the maximum amount of pressure versus the amount of compression, preventing undesirable excessive pressure at higher amounts of compression. In areas of thinner tissue, the resilient layers 130 of multi-layer tissue thickness compensators provide enough thickness to avoid forming a gap between a staple crown and tissue.

An analogy may be drawn to two springs connected in series, one a soft, low-spring constant spring and a second a stiffer, high spring constant spring. In such a system of springs if one spring is substantially stiffer than the other, the stiffness of the softer spring predominates. The system stiffness thus tends to be that of the softer spring.

Considering Figure 4, a soft spring's low spring constant would be illustrated in a simplified, linear sense with a line having a shallow slope. A stiffer spring's high spring constant would be illustrated in a simplified, linear sense with a line having a steeper slope.

Turning to the low compression side to the left of point F in Figure 4 demonstrates function of the construct at low pressure. At low pressure, sacrificial layer 120 is a stiff spring compared to the softer spring of resilient layer 130. It compresses, or, to continue the spring analogy, deflects, little compared to the stiffer resilient layer 130. Since most low-pressure compression occurs in resilient layer 130, compression of the construct created from a combination of sacrificial layer 120 and resilient layer 130 occurs in resilient layer 130 and a stiffness of the combined layer, illustrated by the slope of the low-compression side of curve D, is closer to that of curve B. Resilient layer 130 works advantageously to apply optimum pressure to tissue while relatively uncompressed sacrificial layer 120 fills the gap caused by overly thin or easily compressed tissue.

Turning to the high-compression side to the right of point F in Figure 4 demonstrates the advantage of sacrificial layer 120 in the construct. At high pressure, sacrificial layer 120 becomes a much softer, low spring constant spring, lower than the spring constant of resilient layer 130. The change in properties from a stiff sacrificial layer 120 to a softer sacrificial 120 is illustrated at point F. Because of the change of properties of sacrificial layer 120, the slope of curve D to the right of point F more closely matches the slope curve C than B as the system takes on the properties of the softer spring. Therefore, at high pressures and tissue thicknesses little additional pressure is generated for the additional compression needed by the system. Much more deflection can occur before the pressure reaches a level that is disadvantageous to the tissue. Therefore, the range of tissue thicknesses and compression levels over which construct is available is increased by the addition of sacrificial layer 120 to resilient layer 130.

Materials useful for the construct generally have non-linear pressure-deflection curves, however, designers can design constructs by choosing collapsible materials having useful properties of stiffness at low pressure and collapsibility at higher pressure. Designers can pair these collapsible materials with compatible resilient materials to make the construct useful over a wide range of tissue variables. Additionally, stiffness properties of the construct may be varied advantageously by modifying thicknesses of layers, modifying geometry of layers, modifying the numbers of layers, and by modifying the manufacturing processes creating layers. Although two compressible layers are shown in the above embodiments, three or more layers may also be employed in the construct to obtain specific pressure-deflection curves. For instance, two or more sacrificial layers may be employed, each with a different pressure at which they begin to collapse. Two or more resilient layers may also be employed to adjust the pressure deflection curve as additional compression is achieved.

Figure 10 shows a staple cartridge 135 having an embodiment of tissue compensator 200 attached. Staple cartridge may be, for example, a cartridge described in US patent number 7,934,630 entitled STAPLE CARTRIDGE FOR FORMING STAPLES HAVING DIFFERING FORMED STAPLE HEIGHTS. Tissue compensator 200 has a stiff sacrificial layer 120 lying next to the surface of cartridge 135 through which staples are ejected. One surface of the stiff sacrificial layer 120 may be affixed to the surface of cartridge 135 with adhesive, or with a mechanical attachment. A surface of the second resilient layer 130 affixes to the surface of sacrificial layer 120 opposite the surface attached to the surface of cartridge 135. Staples within cartridge 135 will penetrate the tissue compensator 200 and attach compensator 200 to tissue to be surgically stapled while stapling together layers of tissue. Because of a unique, designed compression curve caused by the combination of sacrificial layer 120 and the resilient layer 130, different tissue thicknesses may be stapled without concern that the formed staple height will be insufficient to apply the appropriate compression to the tissue to prevent leaks or that the formed staple height "h" will over compress the tissue causing it to exhibit necrosis.

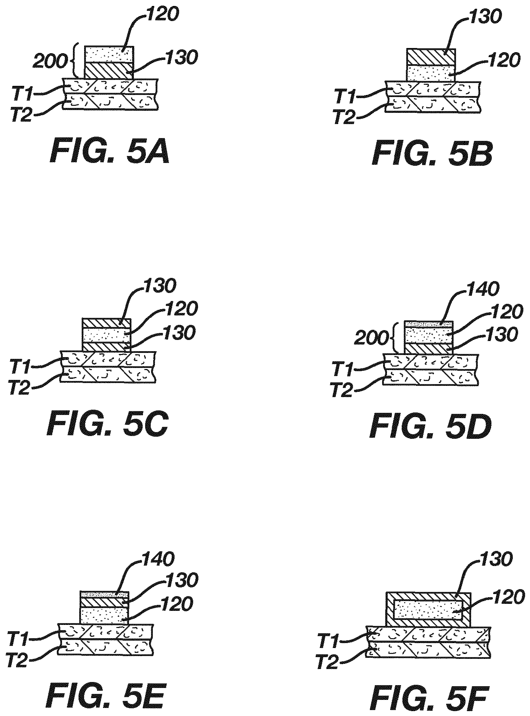

Figures 5A through 5F illustrate configurations of a tissue compensator 200 using the described construct. Figure 5A shows a two-layer tissue compensator 200 comprising resilient layer 130 at the tissue interface and sacrificial layer 120 on top of resilient layer 130. Resilient layer in Figure 5A is situated to distribute load on tissue, while a staple crown may collapse only the portion of sacrificial layer 120 directly under a crown of a staple, embedding the staple crown into sacrificial collapsible layer 120. This configuration may be advantageous if a tissue compensator collapsible at a low force is desirable, for example, to create enough force to hold an attachment into place. Because the area directly under the crown 110 of the staple 10 is small, the pressure to collapse collapsible layer 120 will be reached at a low force level. In an alternative embodiment, foam or solid pledgets such as those shown in U.S. Patent No. 8,499,993 entitled SURGICAL STAPLE CARTRIDGE may be added to the crown 110. The use of these pledgets disperses the staple force on the sacrificial layer 120 over a larger area and reduces the pressure on the sacrificial layer 120.

In another embodiment, shown in Figure 5B, a surface of sacrificial layer 120 is at the tissue interface and resilient layer 130 is adjacent the surface of sacrificial layer 120 opposite the surface of sacrificial layer 120 that is adjacent the tissue. In the configuration of Figure 5B, resilient layer 130 is suited to distribute pressure from the staple crown over a wider area of sacrificial layer 120. Distributing pressure from the staple crown over a wider area collapses collapsible layer 120 at a higher force.

Figure 5C shows collapsible layer 120 sandwiched between two resilient layers 130. These resilient layers 130 abut tissue and a staple crown allowing the force on both the sacrificial layer to be distributed over a larger area and also allowing a resilient layer to be in contact with the tissue.

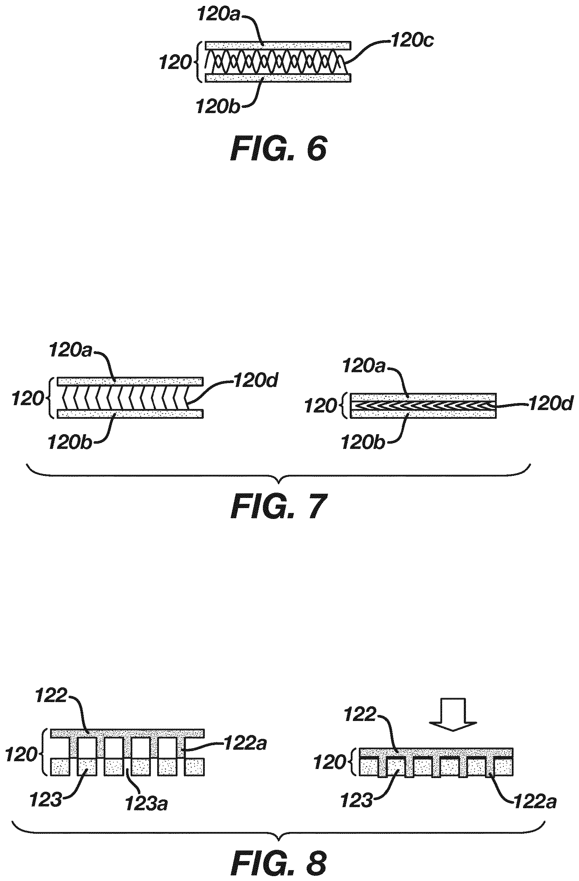

Figures 5D and 5E both show a tissue compensator 200 having a non-collapsible and a non-resilient layer 140 away from tissue and next to a staple crown. The non-collapsible and non-resilient layer 140, may be a plastic film suitable for implantation into a human body, for example, Bionate, manufactured by DSM or SEAMGAURD^{®}, manufactured by GORE^{®}. Non-collapsible and non-resilient layer 140 distributes load from a staple crown over a larger area so sacrificial collapsible layer 120 does not collapse in a limited area under a less than intended force. Figure 5D shows resilient layer 130 adjacent tissue, while Figure 8E shows collapsible layer 120 adjacent tissue.

Figure 5F demonstrates a collapsible layer 120 encased within a resilient layer 130. Resilient layer surrounds collapsible layer 120 on all sides.

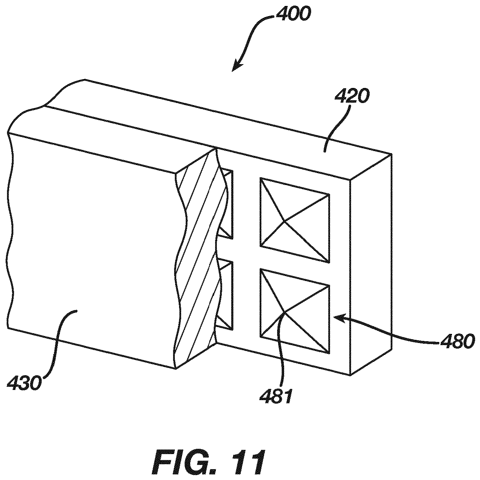

Figure 11 shows other geometries of layers within a tissue compensator 400 which are possible. For example, a tissue compensator 400 could have a pyramidal-shaped collapsible layer 420 sitting atop a resilient layer 430. Such geometry variation can vary the pressure-compression profiles of sacrificial layer 420 and can create advantageous pressure-compression profiles of tissue compensator 400. For the pyramidal layer mentioned above, when a force is applied to the sacrificial layer 420 the pressure at the peak 481 of the pyramid 480 is initially very high due to the small area of the peak 481. As the pyramid 480 compresses or collapses, the contact area of the pyramid 480 increases. In this way the force required to collapse the sacrificial layer 420 increases as the amount of collapse of the sacrificial layer 420 increases.

In certain embodiments, illustrated in Figure 6, sacrificial collapsible layer 120 comprises a multi-layer construct having two or more substantially flat layers 120a and 120b connected by collapsible interposers 120c. Substantially flat layers 120a or 120b can be woven or non-woven, while collapsible interposers 120c can be non-woven. Layers 120 and 130 may also contain hemostatic agents, therapeutic agents, sealants, growth factors, anti-microbial agents, lubricious components and the like. These components and agents may be, for example, silicone, stearates, or glycerol. Other materials and medicaments are described in United States Patent 9,211,120, entitled TISSUE THICKNESS COMPENSATOR COMPRISING A PLURALITY OF MEDICAMENTS.

In one embodiment, a bellows-like or harmonica-like corrugated construct 120d serves as a collapsible interposer. Corrugated construct 120d provides some resistance, but collapses under higher compression, as shown in Figure 7.

In some embodiments illustrated by Figure 8, sacrificial collapsible layer 120 comprises a micro molded or micro-machined construct layer 122 having pins 122a which tightly fit and frictionally engage into apertures 123a of layer 123. Under a higher compression pressure, pins 122a move in apertures 123a collapsing sacrificial collapsible layer 120. At lighter pressures, friction between pins 122a and apertures 123a overcomes the compression pressure and sacrificial collapsible layer 120 remains uncollapsed.

As shown in Figure 9, sacrificial collapsible layer 120 can comprise a fluid-filled shell, or can comprise many fluid-filled shells within collapsible layer 120. These fluid filled shells are resilient under low or medium compression, but burst under higher compression to release the fluid and collapse. The fluid may contain therapeutic agents, and can be encapsulated into closed-cell foam. In some embodiments, the collapse releases the fluid into interstices within the foam. As mentioned above, the fluid filled shells may contain hemostatic agents, therapeutic agents, sealants, growth factors, anti-microbial agents, lubricious components and the like.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DA VINCI^{™} system by Intuitive Surgical, Inc., of Sunnyvale, Calif. Similarly, those of ordinary skill in the art will recognize that various teachings herein may be readily combined with various teachings of U.S. Pat. No. 6,783,524, entitled "Robotic Surgical Tool with Ultrasound Cauterizing and Cutting Instrument," published Aug. 31, 2004.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art. Several of such potential modifications have been mentioned, and others will be apparent to those skilled in the art.

## Claims

1. A fastener cartridge assembly for a surgical instrument, the fastener cartridge assembly comprising:
a. a tissue thickness compensator (200) compressible to a strain level, the tissue thickness compensator comprising:
i. a resilient compensation layer (130) having a first pressure-compression curve;
ii. a collapsible compensation layer (120) adjacent said resilient compensation layer, said collapsible compensation layer having a second pressure-compression curve; and
b. a fastener (10) moveable between an initial position and a fired position, wherein said fastener is configured to compress at least a portion of said tissue thickness compensator when fastener is moved to said fired position,
**characterised in that** a first slope of said second pressure-compression curve is greater than a first slope of said first pressure-compression curve at a low compression and a second slope of said second pressure-compression curve is less than a second slope of said first pressure-compression curve at a compression higher than said low compression.

2. The cartridge assembly of claim 1, wherein the cartridge assembly further comprises a staple cartridge (135) fashioned for ejecting surgical staples into tissue, and wherein the fastener is a surgical staple and said surgical staple is configured to compress at least a portion of said at least one resilient compensation layer when said fastener is moved to said fired position.

3. The cartridge assembly of Claim 2, wherein said collapsible compensation layer is affixed to a surface of said staple cartridge with adhesive.

4. The cartridge assembly of Claim 2 or Claim 3, wherein a surface of said staple cartridge is uneven and said collapsible compensation layer is tailored to fit onto the uneven surface.

5. The cartridge assembly of any one of Claims 1 to 4, wherein said collapsible compensation layer is configured to be compressed at a predetermined pressure.

6. The cartridge assembly of any one of Claims 1 to 5, wherein said collapsible compensation layer is comprised from one of an open celled foam, a closed cell foam or a woven bioabsorbable fabric.

7. The cartridge assembly of Claim 6, wherein said collapsible compensation layer is further comprised of a plurality of liquid filled shells; wherein said liquid filled shells are configured to rupture and release said fluid at a predetermined pressure.

8. The cartridge assembly of Claim 7, wherein said fluid contains one of a hemostatic agent, a therapeutic agent, a sealant, a growth factor, an anti-microbial agent or a lubricating agent.

9. The cartridge assembly of any one of Claims 1 to 8 wherein said collapsible compensation layer comprises two substantially flat layers (120a and 120b) interconnected by collapsible interposers (120c).

10. The cartridge assembly of Claim 9, wherein one of said flat layers further comprises pins (122a) extending perpendicular to said substantially flat surface and wherein second of said flat layers further comprises apertures (123a) for frictionally receiving said pins of said first of said substantially flat layers.

## Patentansprüche

1. Befestigungselementmagazin-Baugruppe für ein chirurgisches Instrument, wobei die Befestigungselementmagazin-Baugruppe umfasst:
a. einen Gewebedickenkompensator (200), der auf ein Verformungsniveau komprimierbar ist, wobei der Gewebedickenkompensator umfasst:
i. eine elastische Kompensationsschicht (130), die eine erste Druck-Kompressions-Kurve aufweist;
ii. eine kollabierbare Kompensationsschicht (120) benachbart zu der elastischen Kompensationsschicht, wobei die kollabierbare Kompensationsschicht eine zweite Druck-Kompressions-Kurve aufweist; und
b. ein Befestigungselement (10), das zwischen einer Ausgangsposition und einer abgefeuerten Position beweglich ist, wobei das Befestigungselement dafür gestaltet ist, wenigstens einen Teil des Gewebedickenkompensators zu komprimieren, wenn das Befestigungselement in die abgefeuerte Position bewegt wird,
**dadurch gekennzeichnet, dass** bei einer geringen Kompression ein erster Anstieg der zweiten Druck-Kompressions-Kurve größer als ein erster Anstieg der ersten Druck-Kompressions-Kurve ist und bei einer Kompression, die größer als die geringe Kompression ist, ein zweiter Anstieg der zweiten Druck-Kompressions-Kurve kleiner als ein zweiter Anstieg der ersten Druck-Kompressions-Kurve ist.

2. Magazinbaugruppe gemäß Anspruch 1, wobei die Magazinbaugruppe ferner ein Klammermagazin (135) umfasst, das zum Ausgeben von chirurgischen Klammern in Gewebe gestaltet ist, und wobei das Befestigungselement eine chirurgische Klammer ist und die chirurgische Klammer dafür gestaltet ist, wenigstens einen Teil der wenigstens einen elastischen Kompensationsschicht zu komprimieren, wenn das Befestigungselement in die abgefeuerte Position bewegt wird.

3. Magazinbaugruppe gemäß Anspruch 2, wobei die kollabierbare Kompensationsschicht mit Klebstoff an einer Oberfläche des Klammermagazins befestigt ist.

4. Magazinbaugruppe gemäß Anspruch 2 oder Anspruch 3, wobei eine Oberfläche des Klammermagazins uneben ist und die kollabierbare Kompensationsschicht dafür zugeschnitten ist, auf die unebene Oberfläche zu passen.

5. Magazinbaugruppe gemäß einem der Ansprüche 1 bis 4, wobei die kollabierbare Kompensationsschicht dafür gestaltet ist, mit einem vorgegebenen Druck komprimiert zu werden.

6. Magazinbaugruppe gemäß einem der Ansprüche 1 bis 5, wobei die kollabierbare Kompensationsschicht eines von einem offenzelligen Schaumstoff, einem geschlossenzelligen Schaumstoff und einem gewebten biologisch absorbierbaren Gewebe umfasst.

7. Magazinbaugruppe gemäß Anspruch 6, wobei die kollabierbare Kompensationsschicht ferner eine Vielzahl von flüssigkeitsgefüllten Schalen umfasst; wobei die flüssigkeitsgefüllten Schalen dafür gestaltet sind, bei einem vorgegebenen Druck zu reißen und das Fluid freizugeben.

8. Magazinbaugruppe gemäß Anspruch 7, wobei das Fluid eines von einem hämostatischen Mittel, einem therapeutischen Mittel, einem Dichtstoff, einem Wachstumsfaktor, einem antimikrobiellen Mittel und einem Schmiermittel enthält.

9. Magazinbaugruppe gemäß einem der Ansprüche 1 bis 8, wobei die kollabierbare Kompensationsschicht zwei im Wesentlichen flache Schichten (120a und 120b), die durch kollabierbare Zwischenstücke (120c) verbunden sind, umfasst.

10. Magazinbaugruppe gemäß Anspruch 9, wobei eine der flachen Schichten ferner Stifte (122a) umfasst, die senkrecht zu der im Wesentlichen flachen Oberfläche stehen, und wobei die zweite der flachen Schichten ferner Öffnungen (123a) zum reibschlüssigen Aufnehmen der Stifte der ersten der im Wesentlichen flachen Schichten umfasst.

## Revendications

1. Ensemble de cartouche d'agrafes pour un instrument chirurgical, l'ensemble de cartouche d'agrafes comprenant :
a. un compensateur d'épaisseur de tissu (200) compressible à un niveau de contrainte, le compensateur d'épaisseur de tissu comprenant :
i. une couche de compensation résiliente (130) ayant une première courbe de pression-compression ;
ii. une couche de compensation repliable (120) adjacente à ladite couche de compensation résiliente, ladite couche de compensation repliable ayant une seconde courbe de pression-compression ; et
b. une agrafe (10) mobile entre une position initiale et une position de déclenchement, ladite agrafe étant configurée pour comprimer au moins une partie dudit compensateur d'épaisseur de tissu lorsque l'agrafe est déplacée à ladite position de déclenchement,
**caractérisé en ce qu'**une première pente de ladite seconde courbe de pression-compression est supérieure à une première pente de ladite première courbe de pression-compression à une faible compression, et une seconde pente de ladite seconde courbe de pression-compression est inférieure à une seconde pente de ladite première courbe de pression-compression à une compression supérieure à ladite faible compression.

2. Ensemble de cartouche selon la revendication 1, l'ensemble de cartouche comprenant en outre une cartouche d'agrafes (135) façonnée pour éjecter des agrafes chirurgicales dans le tissu, et l'agrafe étant une agrafe chirurgicale et ladite agrafe chirurgicale étant configurée pour comprimer au moins une partie de ladite au moins une couche de compensation résiliente lorsque ladite agrafe est déplacée à ladite position déclenchée.

3. Ensemble de cartouche selon la revendication 2, ladite couche de compensation repliable étant fixée à une surface de ladite cartouche d'agrafes avec un adhésif.

4. Ensemble de cartouche selon la revendication 2 ou selon la revendication 3, une surface de ladite cartouche d'agrafes étant irrégulière et ladite couche de compensation repliable étant adaptée pour s'ajuster sur la surface irrégulière.

5. Ensemble de cartouche selon l'une quelconque des revendications 1 à 4, ladite couche de compensation repliable étant configurée pour être comprimée à une pression prédéterminée.

6. Ensemble de cartouche selon l'une quelconque des revendications 1 à 5, ladite couche de compensation repliable étant composée d'une mousse à cellules ouvertes, d'une mousse à cellules fermées ou d'un tissu bioabsorbable.

7. Ensemble de cartouche selon la revendication 6, ladite couche de compensation repliable étant en outre constituée d'une pluralité de coques remplies de liquide ; lesdites coques remplies de liquide étant configurées pour se rompre et libérer ledit fluide à une pression prédéterminée.

8. Ensemble de cartouche selon la revendication 7, ledit fluide contenant un agent hémostatique, un agent thérapeutique, un agent d'étanchéité, un facteur de croissance, un agent anti-microbien ou un agent lubrifiant.

9. Ensemble cartouche selon l'une quelconque des revendications 1 à 8, ladite couche de compensation repliable comprenant deux couches sensiblement plates (120a et 120b) interconnectées par des intercalaires repliables (120c).

10. Ensemble de cartouche selon la revendication 9, l'une desdites couches plates comprenant en outre des broches (122a) s'étendant perpendiculairement à ladite surface sensiblement plate et la seconde desdites couches plates comprenant en outre des ouvertures (123a) pour recevoir par friction lesdites broches de ladite première desdites couches sensiblement plates.
